# EUROPEAN PATENT APPLICATION

(11) **EP 1 393 629 A1**
(43) Date of publication of application: **03.03.2004**
(21) Application number: 02078547.3
(22) Date of filing: 29.08.2002
(51) Int. Cl.: A01N 59/00, A01N 33/12, A61L 2/18, C02F 1/50

(54) **Aqueous disinfecting compositions based on quaternary ammonium monomers**

(71) Applicant: Tevan B.V., 4207 HE Gorinchem-Oost (NL)
(72) Inventor: The designation of the inventor has not yet been filed
(74) Representative: Bird, Ariane

(57) **Abstract**

Aqueous disinfecting liquid compositions comprise hydrogen peroxide, one or more monomeric quaternary ammonium compounds and an amount of water to make up to 100%.

They are characterized in that:
- the weight ratio of hydrogen peroxide to the monomeric quaternary ammonium compounds in the composition is from 8 to 150, and
- the viscosity of the said liquid composition is not above 20 mPa.s.

They are widely applicable to the cleaning, decontaminating, disinfecting or sterilizing a solid surface or a volume of a gas or liquid, for instance by impregnating a carrier.

## Description

The present invention relates to improved aqueous disinfecting, decontaminating, depolluting and cleaning liquid compositions. It further relates to a method for their preparation and to various uses of the said compositions on the one hand in the field of hygiene for cleaning surfaces or treating gas volumes and on the other hand in the field of waste water treatment and pollution management. Finally the invention also relates to disinfecting products comprising a carrier impregnated with such an aqueous liquid composition.

### BACKGROUND OF THE INVENTION

Effective disinfecting processes are necessary for the treatment of bulky amounts of water, especially domestic and industrial circulating waters, and aqueous effluents (such as being present in the foodstuff processing industry) containing microorganisms which cannot be discharged or re-used untreated for hygienic, operational or environmental reasons. Effective disinfecting processes are also necessary for treating surfaces such as premises, equipment, containers, air-conditioning systems and the like. Environmentally compatible disinfecting processes are mainly based on the use of active oxygen compounds, such as hydrogen peroxide

Hydrogen peroxide is a moderately active, mild disinfectant with bactericidal properties. Hydrogen peroxide concentrations of 25 mg/l are known to inhibit the growth of some bacteria, however an effective reduction of the germ count, even at a much higher hydrogen peroxide concentration, takes many hours or requires additional ultraviolet radiation. Generation of the latter, however, requires both expensive equipment and substantial electricity costs. Therefore when disinfecting large amounts of water, for instance for the treatment of water in sewage works and their outputs, such measures are practically inadequate and/or uneconomic. Therefore, various ways to overcome these disadvantages have already been tried in the art.

For instance, U.S. Patent No. 2,917,428 teaches aqueous disinfecting compositions for the treatment of bacteria-containing water, comprising, based on the volume of water to which it is added, from 1 to 1,000 p.p.m. (parts per million) of an alkyl quaternary ammonium salt bactericide, wherein the alkyl radical has less than 20 carbon atoms, and a small but synergistic adjuvant amount, preferably from 1 to 1,000 p.p.m., of hydrogen peroxide. It further teaches that 10 p.p.m. of hydrogen peroxide and 20 p.p.m. of the bactericide, i.e. a synergistic combination with a hydrogen peroxide/bactericide ratio of 0.5, is quite effective. Table III of this document shows that after 5 minutes treatment, a combination of 10 p.p.m. hydrogen peroxide and 2.5 p.p.m. of Arquad T-2C (a mixture of 1 part tallow trimethylammonium chloride and 1 part di-coconut dimethylammonium chloride in a 50% solution in propanol), i.e. a combination with a hydrogen peroxide/alkyl quaternary ammonium salt ratio of 4 provides a log reduction of bacteria of 2.22, whereas a similar combination with a hydrogen peroxide/alkyl quaternary ammonium salt ratio of 2 provides a log reduction of bacteria of 3.0. This means, consistently with the moderate disinfecting activity of hydrogen peroxide, that for a better disinfecting efficiency the hydrogen peroxide amount in the combination should be as low as possible.

U.S. Patent No. 4,073,888 discloses that certain quaternary ammonium salts, being dimethyl dialkyl ammonium chlorides or bromides wherein the carbon atoms in the said alkyl radicals should total 18-24, are effective when used with chlorine dioxide as sanitizing and sterilizing compositions. The examples provided in this document illustrate chlorine dioxide/ ammonium salt ratios from 1.8 to 3.0.

U.S. Patent No. 4,654,208 and U.S. Patent No. 4,499,077 both disclose anti-microbial compositions and methods based on an aqueous solution of a polymeric quaternary ammonium compound (the latter being defined as having a molecular weight of at least about 800) and an oxidizing agent including hydrogen peroxide. The illustrated hydrogen peroxide/ammonium compound ratios are in the range from 0.5 to 2.0.

U.S. Patent No: 4,941,989, U.S. Patent No. 4,847,089 and U.S. Patent No. 5,620,527 all disclose disinfecting compositions and methods based on the synergistically enhanced properties obtained by combining a quaternary ammonium salt and a per-salt. Quaternary ammonium salts are disinfectants and decontaminants themselves, whereas per-salts alone are also known, according to these documents, as excellent disinfectants being superior to hydrogen peroxide in this regard. The per-salts used therein are alkaline water-soluble salts having hydrogen peroxide of crystallization or forms peroxide upon dissociation. When the salts are dissolved in water, peroxide ion is released. Suitable examples are percarbonates, perborates, persilicates and perphosphates associated with a cation such as an alkali metal. Especially preferred is sodium percarbonate having the empirical formula 2Na₂CO₃, 3 H₂O₂. Both documents state that:
- per-salts alone are superior to hydrogen peroxide in regard to disinfection,
- hydrogen peroxide is a weak disinfectant and has poor permeability into bacteria,
- when a per-salt is dissolved in water and liberates hydrogen peroxide, the alkaline salt extracts a proton from the liberated hydrogen peroxide forming the hydroperoxide ion which., in contrast to hydrogen peroxide, is a strong disinfectant and is readily permeably into bacteria.
The preferred positively charged quaternary ammonium salts have a chain of 6 to 30 carbon atoms, preferably 8 to 25, on the quaternary nitrogen. The number of carbons on the said nitrogen is critical, because the quaternary ammonium salt must not only be water-soluble but must also possess sufficient lipophilic character to permit it to pass from the aqueous phase into an oily phase. The hydroperoxide ion becomes associated with the quaternary ammonium ion and its negative charge is effectively neutralized according to the equation: R₄N⁺ + O₂H⁻ → [R₄N⁺⁻O₂H]

The resultant lipophilic quaternary ammonium hydroperoxide may then pass from the aqueous phase into an oily phase where the hydroperoxide ion may exert its disinfecting and decontaminating effects.
The cleaning and disinfecting compositions of U.S. Patent No. 4,941,989, which may be formulated as creams, bulk powders, tablets soaps and solutions for use in health care and surgical applications, broadly contain:
- 10 to 90% by weight of an alkaline water-soluble salt containing hydrogen peroxide of crystallization, and
- a positively charged quaternary ammonium salt in a range from a fraction of a percent to 30%.
The examples illustrated in U.S. Patent No. 4,941,989 relate to fluid cream, pasty and powdery compositions which, since hydrogen peroxide of crystallisation accounts for about 11 % by weight of the alkaline percarbonate being used, exhibit H₂O₂/quaternary ammonium salt weight ratios in the range from 0.3 to 2.2 and H₂O₂ contents from 0.5 to 4.5 weight %. The cream or paste or powder suggested for healthcare in U.S. Patent No. 4,941,989 makes use of an alkaline water-soluble salt having hydrogen peroxide of crystallization which is not a readily inexpensive commercially available material and which due to its solid form generates manufacturing and handling disadvantages associated with solids and/or viscous liquids. Careful examination of the examples illustrated in U.S. Patent No. 4,941,989 also teaches the skilled person that the weight ratio of hydrogen peroxide to the quaternary ammonium salt should be decreased when the viscosity of the composition is decreased.

U.S. Patent No. 5,290,805 and U.S. Patent No. 5,413,722 disclose an aqueous solution comprising a biocidal quaternary ammonium compound selected from decylnonyldimethylammonium halides and decylisononyldimethylammonium halides and being present in the solution in an amount from 5 to 90 weight %. In table 4 (example 5) of U.S. Patent No. 5,290,805, the pool half-life circulation of these compounds is quite favorably compared to that of hydrogen peroxide. However there is no suggestion in this document of combining the said quaternary ammonium compounds and hydrogen peroxide.

U.S. Patent No. 5,780,064 discloses an aqueous germicidal composition comprising a copper salt, a quaternary ammonium compound, and a peroxide, wherein the copper salt is present in an amount from 2 to 20 % by weight of the solution, the quaternary ammonium compound is present in an amount from 0.5 to 2% by weight of the solution, and the peroxide is present in an amount from 0.5 to 5% by weight of the solution. The peroxide may be hydrogen peroxide, and the quaternary ammonium compound may be a trialkyl quaternary ammonium salt wherein each alkyl group contains from 1 to 20 carbon atoms.

Thus there is a strong consensus in the art that when a quaternary ammonium compound is combined with hydrogen peroxide or a hydrogen peroxide-containing salt for disinfecting purposes, the ratio of the latter to the quaternary ammonium compound should specifically be in a range from 0.3 to 4.0 and, within this range, should be as low as possible. It is also known in the art to improve disinfecting properties by replacing hydrogen peroxide with a hydrogen peroxide-containing salt, this measure however being detrimental to the manufacturing and handling facility of the resulting composition because of a too high viscosity at the temperature of use (commonly room temperature).

Thus, there is a strong need in the art for free-flowing disinfecting compositions, especially based on an inexpensive and readily available substance like hydrogen peroxide, being able to provide a high disinfecting efficiency, especially as conventionally measured by reduction of germs, for a wide range of germs (including bacteria, fungi, viruses and the like), within a short period of time, without a significant risk of renewed proliferation of germs and while achieving a satisfactory compromise of environmental and safety requirements, such as minimizing odor and irritation of the human skin or mucosa. There is also a need in the art for such disinfecting compositions which meet all current safety national regulations applicable to the handling of their main active ingredients, e.g. hydrogen peroxide, at each step of their preparation or use, for instance during manufacturing, storage or transportation. There is also a need in the art for such disinfecting compositions which due to their physical free-flowing and easily dilutable form can, upon demand, be readily used in a wide range of applications including, but not limiting to, for instance the decontamination of bulky amounts of waste water or, following mere dilution with water, the cleaning, decontamination or sterilization of surfaces such as hospital, laboratory, domestic and industrial premises and equipment, including containers, incubators, swimming pools and the like. All these needs, either individually or in combination, constitute the various goals of the present invention. Further advantages of the invention will readily appear from the disclosure of the following various embodiments.

### SUMMARY OF THE INVENTION

The present invention is based on the quite unexpected finding that, contrary to the well-established consensus in the art, aqueous disinfecting compositions with a high activity against a wide range of germs can be prepared from a combination of hydrogen peroxide and an organic bactericide being lawfully admitted for contact with foodstuffs, while using a very high ratio of hydrogen peroxide to the said organic bactericide in the combination. The present invention is also based on the quite unexpected finding that, contrary to the well-established consensus in the art, it is not required to replace hydrogen peroxide with a hydrogen peroxide-containing salt, with the detrimental consequences of additional costs and of handling viscous or pasty formulations, in order to overcome the well known insufficient disinfecting activity of hydrogen peroxide.

Based on these findings, the present invention relates to aqueous disinfecting liquid compositions comprising hydrogen peroxide, one or more monomeric quaternary ammonium compounds, water and possibly stabilizers and fragrances, the said compositions having a selected viscosity and selected proportions of hydrogen peroxide and the monomeric quaternary ammonium compounds. The present invention also relates to disinfecting products comprising a carrier impregnated with such aqueous liquid compositions.

### DETAILED DESCRIPTION OF THE INVENTION

A first aspect of the present invention therefore consists of an aqueous disinfecting liquid composition comprising hydrogen peroxide, one or more monomeric quaternary ammonium compounds and an amount of water to make up to 100%, characterised in that:
- the weight ratio of hydrogen peroxide to the monomeric quaternary ammonium compounds in the composition is from about 8 to 150, and
- the viscosity of the said liquid composition is not above about 20 mPa.s. The liquid compositions of the invention are easily diluted with water, starting from a composition being highly concentrated in hydrogen peroxide. Therefore, the hydrogen peroxide content in the said composition may suitably be selected within a broad range from about 0.1 to about 50 weight %.

In the present aqueous disinfecting liquid compositions, in order to achieve at least some of the above-mentioned goals and the unexpected technical effects of this invention whatever the hydrogen peroxide content in the above-mentioned broad range, it is critical that the weight ratio of hydrogen peroxide to the monomeric quaternary ammonium compounds in the composition is from about 8 to 150, preferably from 10 to 100, and more preferably from 20 to 50.

In order to ensure both the required disinfecting efficiency and the required viscosity requirement, it is critical that the quaternary ammonium germicide compound being used is a monomeric compound, i.e. preferably has a molecular weight not above about 600. In practice, considering commercially available such compounds, their molecular weight should be selected within a range from about 250 up to about 600. Preferably the monomeric quaternary ammonium compound is a salt, preferably an inorganic salt such as a halide (more preferably a chloride) or any other non-toxic non-interfering anion (such as for instance hydrogen sulfate, nitrate, sulfate or phosphate). Organic salts such as acetates or other carboxylic acid esters may be considered as well. Any monomeric quaternary ammonium compound with some germicidal activity can be used in the compositions of the invention. Particularly useful quaternary ammonium compounds include monoalkyl, dialkyl, trialkyl and tetraalkyl quaternary ammonium salts, wherein each alkyl group contains 1 to 16 carbon atoms and wherein the carbon atoms in the said alkyl groups should total not more than 40, preferably between 14 and 40. The alkyl radicals are preferred to be but not necessarily symmetrical. The quaternary salt needs not be a pure compound, i.e. the alkyl groups can be a mixture of different molecules. The most preferred species are compounds containing two straight chain alkyl groups having 8 to 16 carbon atoms and two lower alkyl groups having 1 or 2 carbon atoms. Suitable examples of such compounds are the salts of dioctyldimethylammonium, didecyldimethylammonium, decylnonyldimethylammonium, decylisononyldimethylammonium, didodecyldimethylammonium, ditetradecyldimethylammonium and octyldodecyldimethylammonium.

Also useful are quaternary ammonium salts containing one or more substituted or unsubstituted aryl groups (preferably containing one benzyl group) and further containing one to three alkyl groups, wherein each said alkyl group contains 1 to 16 carbon atoms and wherein the carbon atoms in the said alkyl groups should total not more than 30, preferably between 14 and 30. While it is preferred that the compound be symmetrical with respect to the alkyl groups, this symmetry is not essential for the effectiveness of the invention. Examples of suitable compounds include the salts of dodecyldimethylbenzylammonium, tetradecyldimethylbenzylammonium and hexadecyldimethylbenzylammonium.

The aqueous disinfecting liquid compositions according to this invention may further include one or more stabilizers, such as a strong inorganic acid, for instance phosphoric acid, nitric acid, sulfuric acid, hydrobromic acid or boric acid or mixtures thereof, namely for the purpose of adjusting the pH of the composition within a range suitable for handling and use. Among inorganic acid stabilizers, phosphoric acid is especially preferred. In practice, the said acid stabilizer is commonly already incorporated in a suitable amount into the commercially available hydrogen peroxide being used. The stabilizer optionally used in this invention may also be an organic carboxylic acid such as tartaric acid, citric acid (or a hydrate thereof), benzoic acid, picolinic acid, nicotinic acid and isonicotinic acid. Mixtures of inorganic and organic acids may be considered as well for this purpose. The said stabilizer(s), when present, should preferably be used in an amount effective for pH adjustment and/or long-term storage stability of the liquid composition.

The aqueous disinfecting liquid compositions according to this invention may also include at least one component selected from the group consisting of surfactants, corrosion inhibitors and fragrances (perfumes), provided that their amounts and physical forms do not result in raising the viscosity of the composition above about 20 mPa.s.

Suitable surfactants for use herein should be other than the above monomeric quaternary ammonium compound and include for instance cationic, non-ionic, amphoteric and zwitterionic surface-active compounds, preferably those suitable for contact with foodstuffs or drinking water at the relevant dose, and mixtures of such compounds.

A wide variety of non-ionic surfactants are potentially useful herein. Non-limiting examples of anionic surfactants include, for instance, those selected from the group consisting of polyethoxylated and/or polypropoxylated glycols, C₈-C₂₀ fatty acid monoesters, sorbitan monopalmitate and the like. Specific examples of suitable amphoteric surfactants include sodium 3-dodecylaminopropionate, sodium 3-dodecylaminopropane sulfonate, N-alkyltaurines and betaines.

In the disinfecting liquid compositions according to the invention, the weight ratio of the said optional surfactant(s) to the hydrogen peroxide is preferably at most about 0.1:1.

Suitable corrosion inhibitors for use herein, which can also act as a stabiliser, should preferably be admissible for contact with foodstuffs and/or for oral administration with drinking water. Exemplary compounds of such corrosion inhibitors include low molecular weight aliphatic phosphonic acids optionally containing carboxyl groups apart from the phosphonic acid groups. These include for instance aminophosphonic acids (such as dimethylaminomethane diphosphonic acid, aminotri-(methylene phosphonic acid, aminoacetic acid-N-di-(methylene phosphonic acid), 1-amino-1-cyclohexylmethane-1,1-diphosphonic acid, 3-aminopropane-1-hydroxyl-1,1-diphosphonic acid and ethylenediamine-tetra-(methylenephosphonic acid), hydroxyethane-diphosphonic acid, phosphonosuccinic acid, 2-phosphonobutane-1,2,4-tricarboxylic acid and diethylene-triaminepenta(methylene-phosphonic acid), as well as their water-soluble salts, particularly their alkali metal salts (such as sodium or potassium), their ammonium salts and their alkanolamine salts (preferably wherein the alkanol has 2 or 3 carbon atoms, such as the mono-, di- or tri-ethanolamine salts) and mixtures thereof. When present, the said corrosion inhibitor should preferably be used in a weight ratio, with respect to the hydrogen peroxide, not above about 0.05.

The suitable fragrance or perfume is well known to those skilled in the art and, when present, should preferably be incorporated into the aqueous disinfecting liquid composition of the invention in an amount of at most about 0.5% by weight of the composition.

Water which forms the main part of the disinfecting liquid composition according to the invention is preferably a purified water, namely a distilled water, demineralized water or deionized water grade, or a mixture thereof with a standard hardness water. The former purified grade preferably means a water having a resistivity greater than about 10⁵ 'Ω/cm and preferably greater than 10⁶ 'Ω /cm.

The pH of the aqueous liquid compositions according to the invention is preferably between about 1.5 and 5, more preferably between about 2 and 4 and may be adjusted or regulated, if necessary, by means of an acid (e.g. phosphoric acid) as previously indicated.

The aqueous liquid compositions of this invention may be manufactured according to various methods. In a first embodiment, all ingredients of the composition may be mixed at the same time. However, any alternative embodiment including two or more steps may be selected as well. For instance, when the composition includes one or more stabilizers, it may be advantageous to mix a suitable amount of the said stabilizers with hydrogen peroxide in a first step, or to use a commercially available hydrogen peroxide grade already containing a stabilizer in suitable amount, and thereafter to mix a suitable amount of the monomeric quaternary ammonium compound with the said stabilized hydrogen peroxide.

In each case, mixing should be effected in a vessel or apparatus made from a material being resistant to hydrogen peroxide and, when present, to the acidic stabilizer. Suitable but non-limiting examples of such materials include glass, polyethylene and polytetrafluoroethylene. The manufacturing method of the invention is preferably performed under atmospheric pressure and within a temperature range from about 10°C to about 40°C, i.e. there is no need for or advantage resulting from providing specific heating means for raising the temperature above room temperature. The above embodiments of the method are applicable to both concentrated formulations and ready-to-use diluted formulations. In the latter case, it is preferred to first prepare a concentrated formulation from a purified water and then to dilute the said concentrated formulation with water immediately before use of the diluted formulation. The water grade to be used for such dilution is not critical to the present invention. It may be a standard hardness water, such as specified in NEN-EN1276 norm (1997), or a purified water grade preferably as indicated herein-above when the added cost of such water grade is consistent with the economic conditions prevailing in the intended specific use of the ready-to-use diluted formulation.

For the sake of clarity, distinction between concentrated formulations and ready-to-use diluted formulations will be based on the hydrogen peroxide content of the said compositions. By definition, a concentrated formulation or composition herein is one including at least about 8% by weight and up to about 50% by weight hydrogen peroxide. By contrast, a ready-to-use diluted formulation or composition is defined herein as one including less than about 8% by weight hydrogen peroxide.

A second aspect of the present invention relates to the use of an aqueous liquid composition such as disclosed above for cleaning, decontaminating, disinfecting or sterilizing a solid surface or a volume of a gas or liquid.

When an aqueous concentrate liquid composition according to the present invention is used as a disinfecting or sterilizing composition, it is typically applied in the following conditions (concentrations and application time):
- when applied to water treatment: at a concentration of preferably at least about 100 ml, preferably not more than 30,000 ml, of the said concentrate composition per volume of 1 m³, during at least 24 hours;
- when dispersed into a gas like air, in a concentration of at least about 4 mg, preferably not more than about 2 g of the said concentrate composition per gas volume of 1 m³, during at least about 5 minutes.

When the aqueous disinfecting liquid composition of the invention is applied onto a solid surface, it is preferred for safety regulations to employ a ready-to-use diluted formulation obtained by mixing preferably at least about 2% by weight, preferably not more than 15% by weight, of the concentrated composition with water, and moisturizing the said solid surface with the diluted formulation obtained during at least 5 minutes until complete wetting of the solid surface is achieved (which, as is known from the skilled person, may depend upon the surface porosity).

As will be understood to those skilled in the art, the preferred amount of the disinfecting liquid composition to be used will widely vary with the type and amount of micro-organisms present on the solid surface or in the liquid or gas to be treated.

With respect to the many uses of the aqueous liquid compositions according to this invention as a disinfectant, the following application methods are more particularly recommended:
- immersion of the product to be treated into the said aqueous composition,
- spraying of the aqueous disinfecting composition onto a solid surface to be treated, and
- incorporation of the aqueous disinfecting (diluted or concentrated) composition into water to be treated (particularly swimming pool water, industrial process water, waste water and the like).

Therefore, the aqueous disinfecting liquid compositions according to the invention are especially useful for:
(a) the disinfection and hygiene of hospital and laboratory premises, industrial premises (such as milk dairies, cheese dairies, malt houses, breweries, facilities for the production of mineral water, wine, spirits, fruit and vegetable juices; greenhouses; cowsheds, hen houses and stables; packaging lines for foodstuffs, drinks or pharmaceuticals; interiors of aeroplanes and boats) and the contents of said premises, especially the equipment or instruments within said premises;
(b) the sterilization of aseptic enclosures such as incubators for premature animals or growing axenic animals;
(c) the treatment of legionella in air-conditioning systems;
(d) the disinfection and hygiene of storage containers (especially silos) and pipelines for conveying liquid or solid products such as foodstuffs (sugar, tea, coffee, cereals, drinks) and animal feed;
(e) the disinfection and hygiene of swimming pools and other balneotherapy equipments (in which case the composition will preferably be surfactant-free);
(f) the disinfection of systems for the production, transport and storage of drinking water (for instance in wells or storage containers), in which case the composition will preferably be surfactant-free; and
(g) the protection of outdoor crops (such as cereals, tomatoes, banana plantations, hydroponic cultures including witloof, seeds, tubercules and the like), by virtue of its bactericidal, fungicidal, sporicidal, virucidal and antiparasitic properties.

Thus the present invention encompasses methods of cleaning, decontaminating, disinfecting or otherwise sterilizing a solid surface, a liquid volume or a gaseous volume by applying the aqueous concentrate or diluted composition disclosed above to the said surface or by dispersing it into the said liquid or gas. In another aspect, the present invention also relates to a disinfecting product comprising a carrier impregnated with an aqueous liquid composition such as disclosed above. The carrier is preferably a solid carrier such as a woven or non-woven textile product or a cellulose product such as for instance a towel. Methods for impregnating such solid carriers with an aqueous composition and packaging products and methods for keeping the humidity of the impregnated solid carrier under common storage conditions are well known to those skilled in the art.

The aqueous disinfecting liquid compositions of this invention have been found to be active against a wide range of gram-positive and gram-negative bacteria, including for instance *Pseudomonas aeruginosa* (e.g. CMCM-2-22 strain), *Pseudomonas cepacia, Enterobacter cloacae, Enterobacter agglomerans, Klebsiella pneumoniae* (e.g. ATCC-10031 strain), *Eschericia coli, Streptococcus faecalis* (e.g. ATCC-10541 strain), *Staphylococcus cohnii Staphylococcus aureus* (e.g. IP 52154 or ATCC-6538 strain), *Bacillus subtilis* (ATCC-19659 strain) (all being customary to hospital bacterial strains), *Enterococcus facium, Enterococcus hirae, Thiobacillus ferrooxidans* (e.g. ATCC 13661 strain), *Lactobacilli, Thermophilic bacilli, Trychophyton interdigitale* (e.g. ATCC-640 strain), *Clostridium sporogenes* (ATCC-3584 strain), *Clostridium perfringens* (ATCC-13124 strain) and the like. The aqueous disinfecting liquid compositions of this invention have also been found to be active against fungi, including for instance *Candida albicans* (e.g. APCC-2091 strain) *Mycobacterium smegmatis* (e.g. IP 7326 strain), *Aspergillus niger* (e.g. 218 IP strain), *Penicillium verrucosum* and the like. The aqueous disinfecting liquid compositions of this invention have also been found to have antiparasitic activity against for instance *Schistosoma haematobium, Schistosoma mansoni* and the like. The aqueous disinfecting liquid compositions of this invention have also been found to have antiviral activity against various viral strains such as for instance orthopoxvirus, human adenovirus, rhinovirus, herpes virus, enterovirus, influenza virus, poliomyelitis virus and the like.

The aqueous disinfecting or decontaminating liquid compositions of the present invention are able to provide bacterial log reductions of at least 5.0 after 5 minutes of use in a disinfecting or decontaminating treatment such as mentioned above, especially when said bacterial activity is measured in accordance with NEN-EN norm 1276.

The aqueous disinfecting liquid compositions of the present invention have many advantages over the existing disinfectants, in particular:
- they provide substantial germicide activity within a short period of time, within broad temperature limits, namely between about 4°C and 60°C,
- they are ready to use immediately after preparation and can be bottled without further waiting time in customary transport and marketing containers (including those made of glass, metals, porcelain, ceramic, plastics and the like),
- at the preferred concentrations, they exhibit no smell or change in smell with time and produce no health impairment or injury, such as skin irritation, for humans or other mammals, and
- the formulations, whether concentrated or not, can be stored for long periods of time (up to years) at temperatures between about 4°C and 25°C, including incidental temperatures up to about 40°C, without exhibiting a substantial decrease in their germicide activity.

The following examples are provided only for the purpose of illustrating some specific embodiments of the present invention and should not be understood as limiting the scope of the invention in any way.

### EXAMPLE 1 - preparation of a concentrate formulation

The following manufacturing steps are performed in a polyethylene vessel and with equipment (e.g. passivated stainless steel stirring apparatus) which are resistant to hydrogen peroxide. The apparatus and equipment is beforehand cleaned well and rinsed with demineralized water with a conductivity below 10 µS/cm.

2500 grams of a 50% by weight hydrogen peroxide is added to 2425 grams of demineralized water with a conductivity below 10 µS/cm. While stirring, there is added, as a stabilizer, 0.5 gram of a diethylene-triaminepenta(methylenephosphonate) sodium salt commercially available from Giovanni Bozzetto under the tradename Sequion 40Na32 as a 33% solution in water and, one minute thereafter, 80.8 grams of didecyldimethylammonium chloride commercially available from Lonza under the tradename Bardac 22 in the form of a solution in water and isopropanol. Finally, the formulation is stirred for about 15 minutes until a transparent homogeneous product is obtained.

As a consequence, the formulation has the following (weight by weight) composition:
- 73.9 % water,
- 25.0 % hydrogen peroxide,
- 0.003 % stabilizer,
- 0.75 % monomeric quaternary ammonium compound, and
- 0.32 % isopropanol.

### EXAMPLE 2 - bactericidal activity of a ready-to-use diluted composition

The bactericidal activity of a ready-to-use diluted composition obtained from the concentrate formulation of example 1 was determined by incubating it with four different bacteria according to:
- the Organisation for Economic Cooperation and Development Principles on Good Laboratory Practice, as published in OECD, ENV/MC/CHEM/(98)17 (1998), and
- the NEN-EN norm 1276 (as revised in 1997).

The concentrate formulation of example 1 was diluted at 6% by volume with hard water, as described in the norm. Bacteria test strains were obtained from commercial suppliers. The stock solutions have been prepared according to supplier instructions, then aliquoted and frozen in a monitored freezer at -70°C. Other materials used were:
- bovine albumin commercially available from Sigma (ref: A7888),
- catalase from bovine liver commercially available from Fulka (Ref: No 60630, with a concentration of 66025 U/mg protein),
- tryptic soy agar (hereinafter referred as TSA) medium commercially available from Bio Trading and specified by the supplier as follows: 15 g tryptone, 5 g soytone, 5 g sodium chloride, 15 g agar and 1000 ml purified water, pH adjusted to 7.1-7.5 after sterilization,
- egg yolk commercially available from Bio Trading, and
- peptone commercially available from Bio Trading and specified by the supplier as follows: peptone 1.0 g, potassium dihydrogen phosphate 3.56 g, disodium hydrogen phosphate 7.23 g, Tween 80 1 ml and 1000 m; purified water.

Bactericidal activity was, in summary, evaluated by the following method: Bovine albumin (3 g/l) and a bacteria suspension were mixed. 8 ml of the 6% diluted composition obtained from the concentrate formulation of example 1 was added and incubated at 20°C for 5 minutes. 1 ml of the mixture was placed in 8 ml of a neutralizer consisting of peptone + 5% egg yolk + 1% catalase and 1 ml of water. After neutralization, the number of CFU (viable aerobic count per plate) was evaluated after 2 days of incubation at 37°C in the TSA medium. The reduction in viability was calculated according to the standard formula. Bactericidal activity measured after 5 minutes treatment with the aqueous disinfecting diluted composition of the invention, expressed as a logarithmic reduction of the bacteria count, is shown in the following table for each bacterial strain:

**TABLE**

| Strain | Log reduction |
|---|---|
| *Escherichia coli* | > 5.4 |
| *Staphylococcus aureus* | > 5.3 |
| *Enterococcus hirae* | > 5.3 |
| *Pseudomonas aeruginosa* | > 5.6 |

## Claims

1. An aqueous disinfecting liquid composition comprising hydrogen peroxide, one or more monomeric quaternary ammonium compounds and an amount of water to make up to 100%, **characterised in that**:
- the weight ratio of hydrogen peroxide to the monomeric quaternary ammonium compounds in the composition is from 8 to 150, and
- the viscosity of the said liquid composition is not above 20 mPa.s.

2. An aqueous disinfecting liquid composition according to claim 1, wherein the hydrogen peroxide content in the said composition is from about 0.1 to 50 weight %.

3. An aqueous disinfecting liquid composition according to claim 1 or claim 2, **characterized in that** the molecular weight of the monomeric quaternary ammonium compound is not above about 600.

4. An aqueous disinfecting liquid composition according to any of claims 1 to 3, **characterized in that** the viscosity of the said liquid composition is in a range from 1 to about 10 mPa.s.

5. An aqueous disinfecting liquid composition according to any of claims 1 to 4, **characterized in that** it further includes one or more stabilizers.

6. An aqueous disinfecting liquid composition according to any of claims 1 to 5, **characterized in that** it further includes one or more corrosion inhibitors.

7. An aqueous disinfecting liquid composition according to any of claims 1 to 6, **characterized in that** it further includes one or more surfactants, the said surfactants being other than the said monomeric quaternary ammonium compounds.

8. An aqueous disinfecting liquid composition according to any of claims 1 to 7, **characterized in that** it further includes one or more fragances.

9. Use of an aqueous disinfecting liquid composition according to any of claims 1 to 8 for cleaning, decontaminating, disinfecting or sterilizing a solid surface or a volume of a gas or liquid.

10. A disinfecting product comprising a carrier impregnated with an aqueous liquid composition according to any of claims 1 to 8.
